# EUROPEAN PATENT SPECIFICATION

(11) **EP 1 675 562 B1**
(45) Date of publication and mention of the grant of the patent: **21.05.2008**
(21) Application number: 03759070.0
(22) Date of filing: 10.10.2003
(51) Int. Cl.: A61K 8/60, A61Q 19/10

(54) **SHAMPOO AND BODY WASH COMPOSITION**
SHAMPOO UND KÖRPERREINIGUNGSMITTEL
COMPOSITION POUR LE LAVAGE DES CHEVEUX ET DU CORPS

(43) Date of publication of application: 05.07.2006
(73) Proprietor: Frenchtop Natural Care Products B.V., 1718 MK Hoogwoud (NL)
(72) Inventor: JONGENS, Cornelis, Pieter, NL-1715 SJ Spanbroek (NL)
(74) Representative: de Lang, Robbert-Jan
(86) International application number: PCT/NL2003/000688
(87) International publication number: WO 2005/034895

(56) References cited:
- EP-A- 0 445 982
- EP-A- 0 875 239
- EP-A- 0 951 898
- WO-A-00/32220
- WO-A-97/30692
- WO-A-99/59540
- US-B2- 6 537 533
- ANONYMOUS: "Haarbalsam" INTERNET ARTICLE, [Online] March 2002 (2002-03), XP002285767 Retrieved from the Internet: <URL:web.archive.org/web/20020325194039/ww w.citrosept.at/o-k-balsam.html> [retrieved on 2004-06-22]
- ANONYMOUS: "Shampoo" INTERNET ARTICLE, [Online] April 2003 (2003-04), XP002285768 Retrieved from the Internet: <URL:web.archive.org/web/20030425135154/ww w.itc-apocare.dk/02shampoo.html> [retrieved on 2004-06-17]
- DATABASE CAPLUS [Online] CHEMICAL ABSTRACTS SERVICE, COLUMBUS, OHIO, US; 2001 TSUZUKI, MASAHIDE ET AL: "Cosmetics containing 1,2-octanediol as bacteriostatic agent" retrieved from STN Database accession no. 2001:123181 XP002285769 & JP 2001 048720 A2 (ASAHI DENKA KOGYO K. K., JAPAN) 20 February 2001 (2001-02-20)
- DATABASE CAPLUS [Online] CHEMICAL ABSTRACTS SERVICE, COLUMBUS, OHIO, US; NAKAGAWA, RYUICHI ET AL: "Cleansing compositions containing sugar esters and amide ether sulfates" retrieved from STN Database accession no. 1998:485517 XP002285770 & JP 10 195480 A2 (LION CORP., JAPAN) 28 July 1998 (1998-07-28)
- DATABASE CAPLUS [Online] CHEMICAL ABSTRACTS SERVICE, COLUMBUS, OHIO, US; 2001 NAKAMURA, MASAMI ET AL: "Transparent conditioning shampoos" retrieved from STN Database accession no. 2001:874352 XP002285771 & JP 2001 335443 A2 (POLA CHEMICAL INDUSTRIES, INC., JAPAN) 4 December 2001 (2001-12-04)
- DATABASE CAPLUS [Online] CHEMICAL ABSTRACTS SERVICE, COLUMBUS, OHIO, US; TANAKA, SHINICHI ET AL: "Shampoo compositions containing polyoxyethylene alkyl ether acetates and sucrose fatty acid esters" retrieved from STN Database accession no. 2001:347043 XP002285772 & JP 2001 131034 A2 (NAKANO SEIYAKU K. K., JAPAN) 15 May 2001 (2001-05-15)
- DATABASE CAPLUS [Online] CHEMICAL ABSTRACTS SERVICE, COLUMBUS, OHIO, US; KANAYA, TOMOKAZU ET AL: "Shampoos" retrieved from STN Database accession no. 1999:201903 XP002285773 & JP 11 079955 A2 (MILBON K. K., JAPAN) 23 March 1999 (1999-03-23)

## Description

The present invention relates to a wash composition, in particular a shampoo and body wash composition.

In practice many different wash compositions are known.

A problem of many known compositions is that at frequent use they result in skin irritation, skin sensitation or drying out of the skin. This applies in particular for compositions containing surfactants such as sodium lauryl sulfate (SLS) and sodium laureth sulfate (SLES).

A further problem with known wash compositions is that they often contain many non-biodegradable ingredients that are a severe burden for aquatic organisms that exhibit fish toxicity.

An even further problem with known compositions is that they often contain diethanolamine based foam boosters and products comparable in chemical reactivity with diethanolamine. The use of diethanolamine may result in the formation of highly carcinogenic nitrosamines.

Furthermore, many wash products contain synthetic dyes, which may result in e.g. skin irritation and allergic reactions.

It is an object of the present invention to avoid or at least reduce one or more of the above problems.

It is a further object of the present invention to provide a non-irritating, biodegradable shampoo and body wash composition which is substantially composed of components of vegetable origin.

To this end, the present invention provides a wash composition, in particular a shampoo and body wash composition according to claim 1, comprising:
- a surfactant;
- a microbiological control agent;
- a rheological additive;
- a conditioning agent; and
- a solubiliser.

It has been surprisingly found that the composition according to the present invention provides excellent cleaning results of the hair and/or skin, without irritation or sensitation of the skin. An important advantage of the present invention is that the composition may be substantially composed of biodegradable components such as defined by the OECD (Organisation for Economic Cooperation and Development; OECD series on the test guideline program, no. 2. Detailed review paper on biodegradability testing, environment monograph no. 98), which is beneficial for the environment.

A further advantage of the composition of the present invention is that the components used - except water - may (and preferably will) be all from vegetable origin.

The wash composition according to the present invention may be any composition suitable for cleaning the body, clothes, etc., wherein during the wash operation the composition comes into contact with the skin of the user. As an example, the composition may be a hand dishwash liquid, a liquid handsoap, a liquid laundry cleaner, etc. Preferably the composition will be a shampoo or a body wash composition.

The microbiological control agent may be any suitable agent or combination of agents to provide protection from excessive microbiological growth (of e.g. gram-positive and gram-negative bacteria, moulds and yeasts), preferably enabling the final consumer product to satisfy the boundary conditions for shelf life (e.g. in the European Community at least three years according to Directive 76/768/EEC, specifying max. 100 CFU/g). The microbiological control agent may be any of the preservatives indicated in *Preservatives for Cosmetics* of D.C. Steinberg, Allured Publishing Corp., ISBN #0-931710-54-5, which is hereby incorporated by reference.

The microbiological control agent should preferably eliminate pathogenic organisms in the composition while in storage, but leave the symbiotic organisms on the skin in peace after application of the composition on the skin, hair or mucous membranes. Further they should preferably not cause adverse reactions on the skin and in the body such as skin irritation. Furthermore the microbiological control agent should preferably not result in undesired metabolites.

The rheological additive may be any agent (or combination thereof) to obtain rheological properties, whereof viscosity and/or thixotropy are exponents. The person skilled in the art will readily select a proper rheological additive(s) and amounts(s) thereof to obtain the desired rheology. Specific reference is made to Rheological Properties of Cosmetics & Toiletries of D. Laba, Marcel Dekker, New York, 1993, ISBN #0-8247-9090-1, which is hereby incorporated by reference. Non-limiting examples are tree & shrub exudates (karaya gum, tragacanth gum, gum Arabic, gum ghatti), seed extracts (guar gum, locust been gum, tamarind seed), seaweed extracts (carrageenan, alginates, agar), fruit extracts (pectins, waxes), grains & roots (corn starch, potato starch, etc), microbial polysaccharides (Xanthan gum, dextran), modified natural products (cellulose derivatives such as hydropropyl cellulose, methylcellulose, hydroxypropyl methylcellulose, cellulose gum, etc.), petroleum based rheological additives such as acrylic/methacrylic acid homo- and copolymers), acrylamide homo- and copolymers, alkylene oxide homo- and copolymers, etc.), and inorganic rheological additives such as clays and amorphous silica's (hydrated silica, fumed silica).

The conditioning agent may be any agent, such as quaternary ammonium derivatives, to compensate for the build up of a negative electric charge to the hair during the washing process, and / or to improve the combing properties (wet and dry), and / or to avoid "fly-away" of the hair and / or to enable proper detangle properties of the hair. In this respect specific reference is made to Hair Care, N. Allured (ed.), Allured Publishing Corp., ISBN #0-931710-47-2, which is hereby incorporated by reference.

The solubiliser may be any agent suitable to dissolve solid or lipid substances in aqueous systems that are not dissolved by the surfactants used. Well-known solubilisers are e.g. PEG-40 an PEG-80 hydrogenated castor oil, PEG-20 sorbitan laurate, PEG-20 sorbitan caprylate/caprate, ethoxylated fatty alcohols such as oleyl alocohol, lauryl alcohol, trideyl alcohol, etc., polyglyceryl esters and ethers, etc. In this respect specific reference is made to Surfactant Encyclopedia of M.M. Rieger, Allured Publishing Corp., ISBN #0-931710-49-9, which is hereby incorporated by reference.

The person skilled in the art will readily understand that the composition according to the present invention will usually contain water and may comprise many further components, such as henna extract, colouring agents, perfumes, vegetable oils (such as jojoba oil, almond oil, wheat germ oil, walnut oil, avocado oil, rice bran oil, macadamia oil, olive oil, etc.), essential oils (e.g. containing volatile terpenoids such as sweet orange oil, grapefruit oil, tea tree oil, rosemary oil, echinacea oil, lemon oil, juniper oil, lemon grass oil, peach oil, etc) and further herbal extracts (such as chamomile, rosemary, rooibos, green tea, seabuckthorn, comfrey, dandelio, hamamelis, gingko biloba, guarana, etc), vitamins and provitamins (such as panthenol [provitamin B%] and retinol [provitamin A]), etc. Preferably, all components of the composition are from vegetable origin.

According to a preferred embodiment of the present invention, the composition has a RBC (Red Blood Cell test) value ≤ 10, preferably ≤ 5.

Hereby the composition has a very low irritancy for the skin. The RBC-test is known in the field and is amongst others used to classify surfactants on their ability to apply for haemolysis of red blood cells. This property has been identified as a means to quantify the irritancy caused by surfactants relative to the readings of the Draize test. The RBC test is performed on fresh calf blood and is according to the official point of view of the European Community considered not to be an animal test. More information on the RBC-test can be found in e.g. Pape, W.J.W., Pfannenbecker, U. and Hoppe, U., Validation of the red blood cell test as an in vitro assay for the rapid screening of irritation potential of surfactants, Molecular Toxicology, 1 (1987), 525-536 and Pape, W.J.W. and Hoppe, U., Standardisation on an in vitro red blood cell test for evaluating the acute cytotoxic potential of tensides, Arzneimittel-Forschung/Drug Research, 40 (I), 4 (1991), 498-502.

The surfactant comprises a mixture of a sodium acyl glutamate, a sodium ethercarboxylate, an alkylpolyglucoside, a sodium cocoamphoacetate, a cocamidopropyl betaine and behenoyl PG-trimonium chloride.

This mixture has shown to be non-irritating for the skin. Furthermore, all these surfactants are from vegetable origin. An important advantage of this embodiment is that the composition contains substantially no SLS and SLES.

Advantageously the microbiological control agent comprises a compound selected from the group consisting of 1,2-dihydroxyoctane, 1-(2-ethylhexyl)glycerol 1,3-dihydroxypentane or a combination thereof.

It has been found that these compounds efficiently eliminate organisms in the composition while in storage, but leave the symbiotic organisms on the skin in peace after application of the composition.

Preferably, the rheological additive comprises a mineral clay, in particular magnesium aluminum silicate, sodium magnesium silicate or a combination thereof.

It has surprisingly found that mineral clays are not irreversibly shear sensitive and are not electrolyte sensitive. Furthermore these clays exhibit a high degree of thixotropy and do not adversely interfere with other components of the composition of the present invention.

Preferably the conditioning agent comprises behenoyl PG-trimonium chloride or a complex formed thereof.

Behenoyl PG-trimonium chloride is fully biodegradable and has a RBC value of zero. Further it has no biocidal properties. In combination with sodium laureth-11 carboxylate it forms a complex which is soluble in mixtures of anionic surfactants.

Advantageously, the solubiliser comprises a sucrose monoester, in particular of a C8-C14 carboxylic acid; a branched or cyclic C8 - C18 carboxylic acid; or a combination thereof.

These solubilisers have shown to be very efficient in solubilising triglyceride based vegetable oils such as avocado oil, walnut oil, etc.; liquid wax esters such as jojoba oils; and essential oils such as lavender oil, tea tree oil, echinacea oil, rosemary oil, etc. which may be present as further components.

In a further aspect the present invention relates to the use of the composition according to the present invention as a shampoo or body wash composition, in particular as a shampoo.

Hereinafter the present invention will be illustrated in more detail by non-limiting examples.

### Example 1

A non-irritating and biodegradable shampoo and body wash composition according to the present invention was prepared as follows, using the following components:
Surfactants: (preferably 8-22 g, more preferably 13-15 g);
Microbiological control agent: (preferably 0.3-1.0 g, more preferably 0.5-0.7 g);
Rheological additive: (preferably 1.5-5 g, more preferably 2.5-3.0 g);
Conditioning agent: (preferably 0.2-1.0 g, more preferably 0.3-0.5 g);
Solubiliser: (preferably 0.4-1.2 g, more preferably 0.7-0.9 g); and
Further additives: (preferably total of 0.5-3.0 g, more preferably 0.8-1.2 g) and water (preferably 60-85 g, more preferably 75-80 g). The above ranges in grams are for 100 g of the composition.

3 g Xanthan gum was dispersed in 197 g water until a homogeneous, high viscous gel (Brookfield viscosity of 2500 - 4500 cPs) was obtained. Subsequently 8 g capryl glycol was added while heating gently to 30-25°C until all capryl glycol had been dissolved and a homogeneous gel had been obtained.

30 g Sodium magnesium silicate was dispersed in 300 g demineralised water using a Silverson L4RT rotor-stator mixer at high speed (about 5000 rpm). After about 20 minutes stirring an almost transparent, bluish, low viscous dispersion (Brookfield viscosity of 20-40 cPs) was obtained (particle size was about 20 nm).

8 g Behenoyl PG-trimonium chloride was dissolved in 40 g sodium laureth-11 carboxylate/laureth-10 at elevated temperature (35-40°C); a molecular complex was formed that was fully transparent and high viscous (Brookfield viscosity of 700-1000 cPs).

100 g Cocamidopropyl betaine was added to the xanthan gum gel followed by a homogenisation step using a Silverson L4RT rotor-stator mixer at low speed (about 250 - 1000 rpm). After homogenisation the other surfactants were subsequently added while stirring with a planetary mixer: 70 g sodium cocoyl glutamate, 50 g sodium cocoamphoacetate, 40 g decyl polyglucose, 10 g isostearamide MIPA, and 15 g betaine. The obtained low-viscous mixture (Brookfield viscosity of 400 - 800 cPs) was stirred for 15 minutes.

The above formed molecular complex was added to the surfactant mixture and stirred with a planetary mixer until completely absorbed in the surfactant mixture. The obtained mixture was stirred during 30 minutes.

The aqueous dispersion of sodium magnesium silicate was added to the surfactant mixture. After a few minutes the viscosity of the surfactant mixture markedly increased. Stirring was continued for 15 minutes. The product was highly thixotropic; the viscosity further increased as soon as stirring was discontinued.

A solution of 5 g panthenol in 50 g demineralised water was added and the product was again stirred with a planetary mixer for 15 minutes.

A premix was prepared by dissolving 25 g sucrose laurate in 30 g water. Then additional ingredients such as botanical extracts (15 g of e.g. chamomile, guarana, green tea, henna, rooibos, rosemary, orange flowe, echinacea, hop, horse chestnut); vegetable oils and/or liquid waxes (20 g) such as avocado oil, walnut oil, wheat germ oil, jojoba oil, etc.; 0,05 - 0,2 g of essential oils (such as echinacea oil, peppermint oil, rosemary oil, tea tree oil, eucalyptus oil, etc.) or vegetable natural dyes (e.g. lactoflavine/riboflavine, betanine, cochenille, indigo, carotenoides, xantophylls, anthocyanines); were subsequently dissolved under slight heating until 35°C until a homogeneous translucent/opaque micro-emulsion was formed. This premix was added to the above surfactant composition. After the addition stirring was continued with a planetary mixer during 10 minutes. Finally the pH was adjusted to 5,5 using either a 50% aqueous citric acid solution or an 18% aqueous sodium hydroxide solution.

The RBC value of the composition was determined and was 4,8.

### Example 2

The following composition was prepared in analogy to the method of preparation of Example 1. The following components were used.
Surfactants: sodium cocoyl glutamate (3 g), sodium laureth-11 carboxylate/laurth-10 (6 g), sodium cocoamphoacetate (4 g), decyl polyglucose (3 g), cocamidopropyl betaine (3 g), isostearamide MIPA (0.8 g);
Microbiological control agent: capryl glycol (0.6 g);
Rheological additive: sodium magnesium silicate (2.5 g);
Conditioning agent: behenoyl PG-trimonium chloride (0.5 g);
Solubiliser: sucrose laurate (0.8 g); and
Further additives: panthenol, rooibos extract, chamomile, green tea extract, henna extract, jojoba extract, avocado oil, rice bran oil (total of 1.6 g) and water (74.2 g).

The RBC value of the composition according to Example 2 was determined and was 4,6.

### Example 3

The following composition was prepared in analogy to the method of preparation of Example 1. The following components were used.
Surfactants: sodium cocoyl glutamate (3 g), sodium laureth-8 carboxylate/laureth-7 (5.5 g), disodium cocamphodiacetate (3.8 g), lauryl polyglucose (4.2 g), cocamidopropyl betaine (3.2 g), isostearamide MIPA (0.8 g);
Microbiological control agent: capryl glycol (0.5 g), 1,3-pentylene glycol (2.2 g);
Rheological additive: sodium magnesium silicate (2.7 g);
Conditioning agent: behenoyl PG-trimonium chloride (0.5 g);
Solubiliser: sucrose caprylate (0.5 g), sucrose caprate (0.5 g); and

Further additives: panthenol, rooibos extract, hop extract, guarana extract, henna extract, jojoba oil, peach kernel oil, walnut oil, glycerin (total of 3.5 g) and water (69.1 g).

The RBC value of the composition according to Example 3 was determined and was 4,8.

### Example 4

The following composition was prepared in analogy to the method of preparation of Example 1. The following components were used.
Surfactants: sodium lauroyl glutamate (3 g), disodium laureth sulfosuccinate (5.5 g), sodium cocoamphoacetate (4 g), lauryl polyglucose (3 g), cocamidopropyl sultaine (3 g), isostearamide MIPA (0.8 g);
Microbiological control agent: capryl glycol (0.5 g), octoxyglycerin (2 g);
Rheological additive: sodium magnesium silicate (2.8 g);
Conditioning agent: behenoyl PG-trimonium chloride (0.4 g);
Solubiliser: sucrose laurate (0.8 g); and

Further additives: panthenol, horse chestnut extract, orange flower extract, henna extract, jojoba oil, avocado oil, wheat germ oil, glycerin (total of 4 g) and water (70.2 g).

The RBC value of the composition according to Example 4 was determined and was 4,5.

### Example 5

The following composition was prepared in analogy to the method of preparation of Example 1. The following components were used.
Surfactants: sodium cocoyl glutamate (3.5 g), disodium laureth sulfosuccinate (3 g), disodium cocamphodiacetate (4 g), decyl polyglucose (3.6 g), cocamideopropyl sultaine (2.8 g), isostearamide MIPA (0.8 g);
Microbiological control agent: capryl glycol (0.5 g), octoxyglycerin (2 g);
Rheological additive: sodium magnesium silicate (2.8 g);
Conditioning agent: behenoyl PG-trimonium chloride (0.5 g);
Solubiliser: sucrose caprylate (0.5 g), sucrose caprate (0.5 g); and

Further additives: panthenol, horse chestnut extract, orange flower extract, henna extract, jojoba oil, avocado oil, walnut oil, glycerin, propylene glycol (total of 5.2 g) and water (70.3 g).

The RBC value of the composition according to Example 5 was determined and was 4,6.

## Claims

1. Wash composition, in particular a shampoo or body wash composition, comprising:
- a surfactant comprising a mixture of a sodium acyl glutamate, a sodium ethercarboxylate, an alkylpolyglucoside, a sodium cocoamphoacetate, a cocamidopropyl betaine and behenoyl PG-trimonium chloride,
- a microbiological control agent;
- a rheological additive;
- a conditioning agent; and
- a solubiliser.
wherein the composition has a RBC value ≤ 5.

2. Composition according to the preceding claim, wherein the microbiological control agent comprises a compound selected from the group consisting of 1,2-dihydroxyoctane, 1-(2-ethylhexyl)glycerol 1,3-dihydroxypentane or a combination thereof.

3. Composition according to any of the preceding claims, wherein the rheological additive comprises a mineral clay, in particular magnesium aluminum silicate, sodium magnesium silicate or a combination thereof.

4. Composition according to any of the preceding claims, wherein the solubiliser comprises a sucrose monoester, in particular of a C8 - C14 carboxylic acid; a branched or cyclic C8 - C18 carboxylic acid; or a combination thereof.

5. Use of the composition according to any of the preceding claims as a wash composition selected from the group consisting of a hand dishwash liquid, a liquid handsoap, a liquid laundry cleaner, a shampoo and a body wash composition, preferably a shampoo or body wash composition.

## Patentansprüche

1. Reinigungsmittel-Zusammensetzung, insbesondere ein Shampoo oder ein Körperreinigungsmittel,
**gekennzeichnet durch**
- ein Tensid, das eine Mischung aus Natrium-Acylglutamat, Natrium-Ethercarboxylat, Alkylpolyglucosid, Natrium-Cocoamphoacetat, Cocamidopropyl Betain und Behenoyl PG-Trimonium Chlorid umfaßt sowie
- einen mikrobiologischen Wirkstoff,
- ein Bindemittel,
- ein Konditionierungsmittel und
- ein Lösemittel,
wobei der RBC-Wert der Zusammensetzung ≤ 5 ist.

2. Zusammensetzung nach Anspruch 1,
**dadurch gekennzeichnet, daß**
der mikrobiologische Wirkstoff eine Verbindung aus der Gruppe umfaßt, die aus 1,2-Dihydroxyoctan, 1- (2-Ethylhexyl) Glycerol, 1,3-Dihydroxypentan oder aus einer Kombination hieraus besteht.

3. Zusammensetzung nach Anspruch 1 oder 2,
**dadurch gekennzeichnet, daß**
das Bindemittel ein Tonmineral, insbesondere Magnesium-Aluminumsilikat, Natrium-Magnesium-Silikat oder eine Kombination hieraus umfaßt.

4. Zusammensetzung nach einem der vorhergehenden Ansprüche,
**dadurch gekennzeichnet, daß**
das Lösemittel ein Saccharosemonoester, insbesondere aus einer C8-C14 Carbonsäure, einer verzweigten oder cyclischen C8-C18 Carbonsäure oder einer Kombination hieraus umfaßt.

5. Verwendung der Zusammensetzung nach einem der vorhergehenden Ansprüche als Reinigungsmittel aus der Gruppe die Handgeschirrspülmittel, flüssige Handseifen, flüssige Waschmittel, Shampoo und Körperreinigungsmittel, vorzugsweise Shampoo oder Körperreinigungsmittel umfaßt.

## Revendications

1. Composition de lavage, en particulier un shampooing ou une composition de savon liquide pour le corps, qui comprend :
- un surfactant comprenant un mélange d'un acyl glutamate de sodium, d'un éthercarboxylate de sodium, d'un alkylpolyglucoside, d'un cocoamphoacétate de sodium, d'une cocamidopropyl bétaïne et de chlorure de béhénoyl PG-trimonium ;
- un agent de lutte microbiologique ;
- un additif rhéologique ;
- un agent de conditionnent ; et
- un solubilisant,
dans laquelle la composition a une valeur de RBC ≤ 5.

2. Composition selon l'une quelconque des revendications précédentes, dans laquelle l'agent de lutte microbiologique comprend un composé choisi dans le groupe constitué par le 1,2-dihydroxyoctane, le 1,3-dihydroxypentane de 1-(2-éthylhexyl) glycérol ou une combinaison de ceux-ci.

3. Composition selon l'une quelconque des revendications précédentes, dans laquelle l'additif rhéologique comprend une argile minérale, notamment du silicate d'aluminium et de magnésium, du silicate de sodium et de magnésium ou une combinaison de ceux-ci.

4. Composition selon l'une quelconque des revendications précédentes, dans laquelle le solubilisant comprend un monoester de sucrose, en particulier d'un acide carboxylique en C8-C14 ; un acide carboxylique en C8-C18 ramifié ou cyclique ; ou une combinaison de ceux-ci.

5. Utilisation d'une composition selon l'une quelconque des revendications précédentes, en tant qu'une composition de lavage choisie dans le groupe constitué par un liquide pour la vaisselle à la main, un savon liquide pour les mains, une lessive liquide pour le linge, un shampooing et une composition de savon pour le corps, de préférence un shampooing ou une composition de savon pour le corps.
